# EUROPEAN PATENT APPLICATION

(11) **EP 4 309 612 A1**
(43) Date of publication of application: **24.01.2024**
(21) Application number: 22770244.6
(22) Date of filing: 14.02.2022
(51) Int. Cl.: A61B 34/30, B25J 9/16, B25J 13/00

(54) **CONTROL METHOD FOR SURGICAL ROBOT SYSTEM, READABLE STORAGE MEDIUM, AND ROBOT SYSTEM**

(30) Priority: 16.03.2021 CN 202110283035
(71) Applicant: Shanghai Microport Medbot (Group) Co., Ltd., Shanghai 201203 (CN)
(72) Inventor: LI, Zihan, Shanghai 201203 (CN); HE, Chao, Shanghai 201203 (CN); WANG, Xuanhui, Shanghai 201203 (CN); WANG, Jiayin, Shanghai 201203 (CN); LIAO, Zhixiang, Shanghai 201203 (CN)
(74) Representative: Patentship Patentanwaltsgesellschaft mbH
(86) International application number: PCT/CN2022/076250
(87) International publication number: WO 2022/193889

(57) **Abstract**

The present invention provides a control method for a surgical robot system, a readable storage medium and a robot system. The control method for the surgical robot system includes: configuring a corresponding response to malfunction according to instrument information of different surgical instruments; monitoring a state of the surgical robot system, and determining the response to malfunction for a current surgical instrument according to the instrument information of the current surgical instrument when the surgical robot system is in a malfunction state; and controlling the surgical robot system to perform a responsive operation according to the determined response to malfunction. In this way, according to the instrument information of different surgical instruments, the surgical robot system can determine different responses to malfunction and perform corresponding responsive operations, which improves the safety of surgical operations performed by the surgical robot system.

## Description

### TECHNICAL FIELD

The present invention relates to the field of simulation-assisted surgical systems and methods, in particular to a control method for a surgical robot system, a readable storage medium, and a robot system.

### BACKGROUND

The emergence of surgical robots is in line with the development trend of precision surgery. Surgical robots become a powerful tool to help doctors to complete operations. For example, *da Vinci^{™}* surgical robots have been used in major hospitals around the world, bringing benefits to patients because of their advantages of less harm to patients, less bleeding, and faster recovery.

At present, the surgical robot system will switch to the safety protection mode regardless of a recoverable malfunction or an unrecoverable malfunction during the operation, and all motor drivers will be locked and disabled to prevent the surgical robot from continuing to move and causing harm to the patient. However, when the surgical robot system is in the safety protection mode, the control system of the surgical robot also fails to control the posture and clamping state of the tailing end of the instrument, which may introduce surgical risks in the following scenarios;
1) When a malfunction occurs, the surgical instrument is clamping the blood vessel to stop bleeding. In this scenario, disabling the instrument control motor will cause the surgical instrument to lose its clamping force, resulting in blood vessel bleeding and introducing surgical risks;
2) When a malfunction occurs, the surgical instrument is pulling vital organs such as the prostate and liver. In this scenario, disabling the instrument control motor will cause the surgical instrument to lose its clamping force, causing vital organs and tissues to slide freely uncontrollably, easily colliding with other surgical instruments during the operation, and causing tissue scratches, or causing the surgical instrument to change the posture of its tailing end under the action of gravity, and the tailing end of the instrument to move uncontrollably, which may scratch the tissue;
3) When a malfunction occurs, the surgical instrument is holding the suture needle. In this scenario, disabling the instrument control motor will cause surgical instrument to drop needle and introduce surgical risks;
4) When a network communication failure occurs, the control system of the surgical robot loses contact with the instrument driver. In this scenario, the posture and clamping state of the tailing end of the surgical instrument are not controllable, which introduces surgical risks.

### SUMMARY OF THE INVENTION

An object of the present invention is to provide a control method for a surgical robot system, a readable storage medium and a surgical robot system, so as to solve the problem that an existing surgical robot system may lose control of the posture and clamping state of its tailing end when in the safety protection mode, which may introduce surgical risk.

In order to solve the above technical problems, according to a first aspect of the present invention, it provides a control method for a surgical robot system, comprising:
Step S 1: configuring a corresponding response to malfunction according to instrument information of different surgical instruments;
Step S2: monitoring a state of the surgical robot system, and determining the response to malfunction for a current surgical instrument according to the instrument information of the current surgical instrument when the surgical robot system is in a malfunction state; and,
Step S3: controlling the surgical robot system to perform a responsive operation according to the determined response to malfunction.

Optionally, the instrument information comprises state information of the surgical instrument, and wherein determining the response to malfunction for a current surgical instrument according to the instrument information of the current surgical instrument in Step S2 comprises:
determining the response to malfunction for a current surgical instrument according to the state information of the surgical instrument;
wherein the response to malfunction comprises: switching the surgical instrument to a state maintenance mode, and switching the surgical instrument not to the state maintenance mode but to a safety mode.

Optionally, the instrument information further comprises type information of the surgical instrument; the type information of the surgical instrument indicates a dual headed instrument or a single headed instrument; if the type information of the surgical instrument indicates a dual headed instrument, the state maintenance mode comprises simultaneously maintaining a posture of a tailing end of the surgical instrument and a clamping state of the surgical instrument; if the type information of the surgical instrument indicates a single headed instrument, the state maintenance mode comprises maintaining the posture of the tailing end of the surgical instrument.

Optionally, maintaining the posture of the tailing end of surgical instrument comprises at least one of the following:
performing posture maintenance according to a current posture of the surgical instrument, or
performing posture maintenance according to a posture command of the surgical instrument, or
performing posture maintenance by tracking a current position of the surgical instrument in Cartesian coordinate system.

Optionally, the state information of the surgical instrument comprises installation information indicating whether the surgical instrument is installed, and/or enabling information indicating whether the instrument driver is enabled.

Optionally, when it is detected that the installation information indicates that the surgical instrument is installed and the enabling information indicates an enabling state, the response to malfunction comprises switching the surgical instrument to the state maintenance mode;
when it is detected that the installation information indicates that the surgical instrument is not installed, or the enabling information indicates a disabling state, the response to malfunction comprises switching the surgical instrument to the safety mode.

Optionally, the control method for the surgical robot system further comprises:
Step S0: determining a malfunction classification according to a predetermined regulation based on a malfunction information received when the surgical robot system is in a malfunction state;
Step S4: after performing the responsive operation in Step S3, prompting recovery information corresponding to the malfunction classification according to the malfunction classification.

Optionally, the malfunction classification comprises recoverable malfunction and unrecoverable malfunction;
prompting recovery information corresponding to the recoverable malfunction comprises prompting a fault code for an operator to perform a cancelling operation;
prompting recovery information corresponding to the unrecoverable malfunction comprises prompting restart information for the operator to perform a restart operation.

Optionally, the control method for the surgical robot system further comprises:
detecting a state of a communicative connection between the instrument driver and the control device; if the state of the communicative connection is abnormal, the instrument driver performs the control method for the above surgical robot system; if the state of the communicative connection is normal, the control device performs the control method for the above surgical robot system.

In order to solve the above technical problems, according to a second aspect of the present invention, it provides a readable storage medium, storing a program, wherein
when the program is executed, the above control method for the surgical robot system is performed.

In order to solve the above technical problems, according to a third aspect of the present invention, it provides a surgical robot system, comprising: a patient-side operating table and a control device;
wherein the patient-side operating table comprises a mechanical arm to which a surgical instrument is mounted or connected; the control device is communicatively connected to the surgical instrument;
wherein the surgical robot system is configured such that:
   when the surgical robot system is in a malfunction state, the control device performs a responsive operation according to the above control method for the surgical robot system; or
   according to the above control method for the surgical robot system, the state of the communicative connection between the instrument driver and the control device is detected, and the device driver or the control device is selected to perform a responsive operation, depending on whether the state of the communicative connection is normal or abnormal.

In conclusion, in the control method for the surgical robot system, the readable storage medium and the surgical robot system according to the present invention, the control method for the surgical robot system includes: configuring a corresponding response to malfunction according to the instrument information of different surgical instruments; monitoring a state of the surgical robot system, and determining the response to malfunction for a current surgical instrument according to the instrument information of the current surgical instrument when the surgical robot system is in a malfunction state; and controlling the surgical robot system to perform a responsive operation according to the determined response to malfunction. With such a configuration, according to the instrument information of different surgical instruments, the surgical robot system can determine different responses to malfunction and perform corresponding responsive operations, which improves the safety of surgical operations performed by the surgical robot system.

### BRIEF DESCRIPTION OF THE DRAWINGS

Those of ordinary skill in the art will understand that the provided drawings are for better understanding of the present invention, but do not constitute any limitation to the scope of the present invention.
Fig. 1 is a schematic diagram showing an application of a surgical robot system according to an embodiment of the present invention;
Fig. 2 is a schematic diagram showing a surgical robot system according to an embodiment of the present invention;
Fig. 3 is a flowchart of a control method for a surgical robot system according to an embodiment of the present invention;
Figs. 4a-4c are schematic diagrams showing a tailing end of a surgical instrument according to an embodiment of the present invention;
Fig. 5 is a flowchart of a control method for a surgical robot system with a unrecoverable malfunction according to an embodiment of the present invention;
Fig. 6 is a flowchart of a control method for a surgical robot system with a recoverable malfunction according to an embodiment of the present invention;
Fig. 7 is a flowchart of a control method for a surgical robot system according to another embodiment of the present invention;
Fig. 8 is a schematic diagram showing the detection on the state of communicative connection according to an embodiment of the present invention.

In the drawings:
10, doctor-side console; 20, image trolley; 30, patient-side operating table; 31, mechanical arm; 40, surgical instrument; 41, dual headed instrument; 411, pitch joint; 412, yaw joint; 413, rotation joint; 414, clamping piece; 50, hospital bed; 60, auxiliary equipment; 70, beat wave signal; 71, control device; 72, instrument driver.

### DETAILED DESCRIPTION

To make the objectives, advantages and features of the present invention clearer, the present invention will be further described in detail below with reference to the figures. It is noted that the figures are provided in a very simplified form not necessarily presented to scale, with their only intention to facilitate convenience and clarity in explaining the disclosed embodiments. In addition, the structures shown in the drawings are often a part of the actual structures. In particular, the figures generally give emphasis on different details and are accordingly drawn to different scales.

As used herein and in the appended claims, the singular forms of "a", "an", and "the", include plural references unless the context clearly dictates otherwise. In addition, the term "or" is generally used as having the meaning including "and/or", unless otherwise explicitly stated. The term "several" is generally used in the sense including "at least one". The term "at least two" is generally used in the sense including "two or more". In addition, the terms "first", "second", and "third" are used for descriptive purposes only, and cannot be interpreted as indicating or implying relative importance or implicitly specifying the quantity of the indicated technical features. Thus, a feature defined as "first", "second" and "third" may explicitly or implicitly include one or at least two of these features. The term "proximal" is generally the end closer to the operator and the term "distal" is generally the end closer to the patient. "One end" and "the other end" and "proximal end" and "distal end" generally refer to the corresponding two parts and not only include the endpoints, unless the content clearly indicates otherwise. As used in the present invention, unless otherwise clearly stated, the terms "installation", "connection" and "couple" should be interpreted in a broad sense, for example, it may be a fixed connection, a detachable connection, or an integral body; it may be a mechanical connection or an electrical connection; it may be a direct connection or an indirect connection through an intermediary, and it may be an internal communication between two components or an interaction relationship between two components. In addition, as used in the present invention, arranging an element on another element usually only means that there is a connection, coupling, cooperation or transmission relationship between the two elements, and the relationship between the two elements can be directly established or indirectly established through an intermediate element by connection, coupling, cooperation or transmission, and cannot be understood as indicating or implying the spatial positional relationship between two elements, that is, one element can be in any orientation such as inside, outside, above, below or on one side of another element, unless the content is clearly stated otherwise. Those of ordinary skill in the art can understand the specific meanings of the above terms in the present invention according to specific situations.

An object of the present invention is to provide a control method for a surgical robot system, a readable storage medium and a surgical robot system, so as to solve the problem that an existing surgical robot system may lose control of the posture and clamping state of its tailing end when in the safety protection mode, which may introduce surgical risk.

It will be described below with reference to the accompanying drawings.

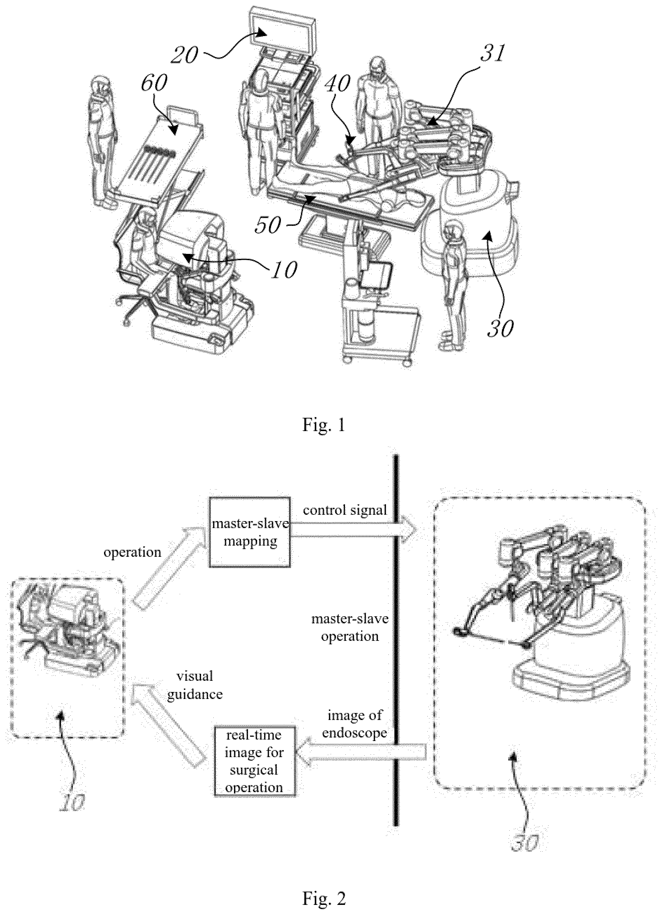

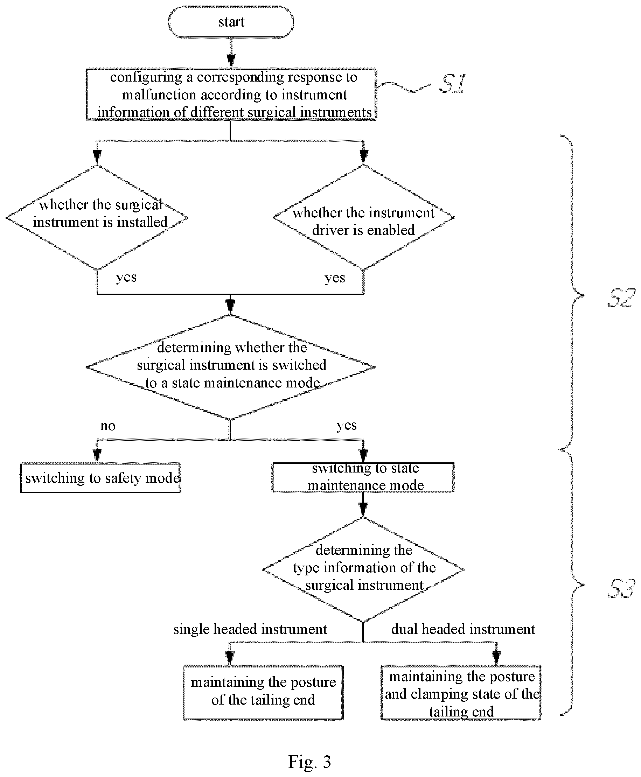

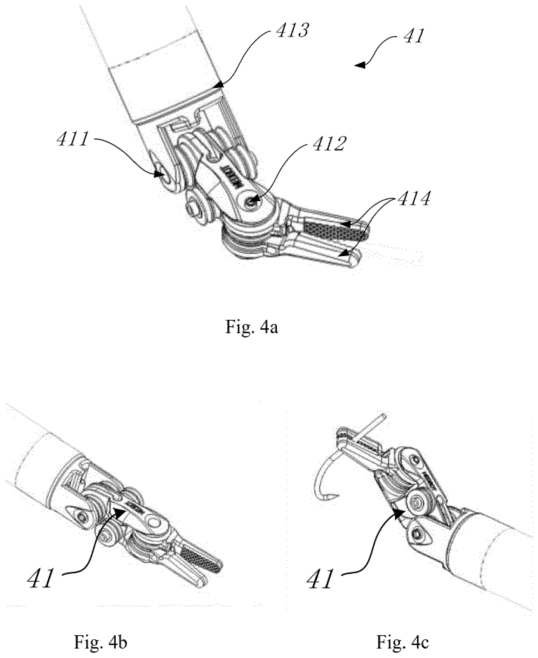

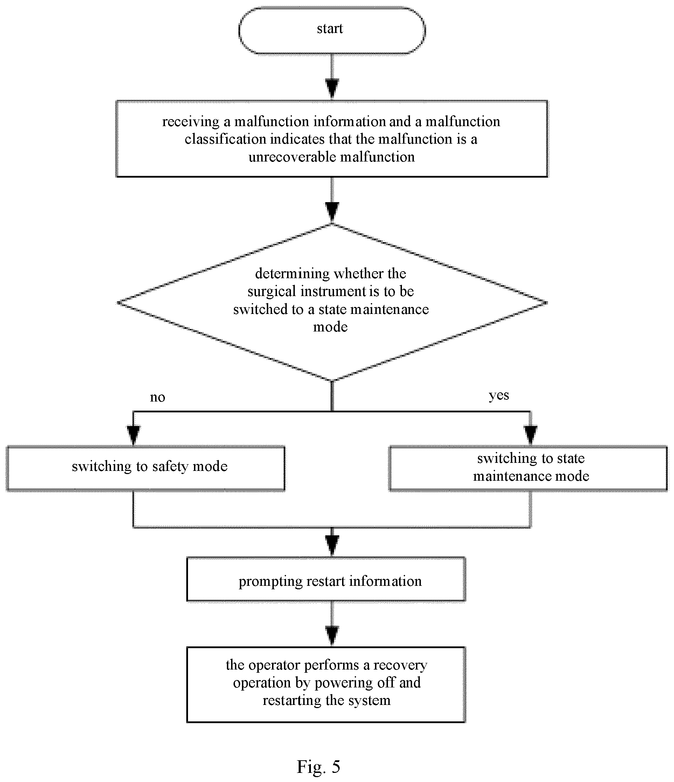

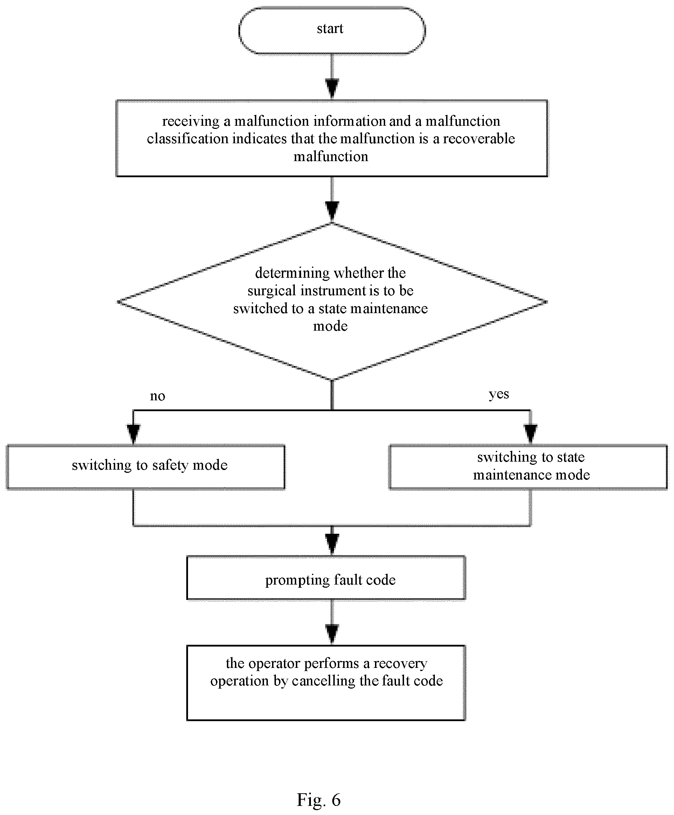

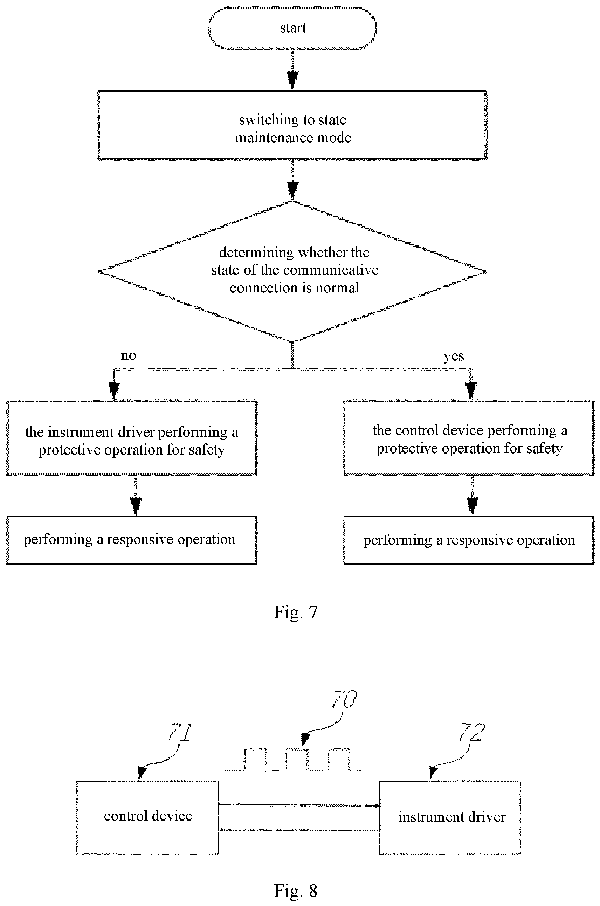

Please refer to Figs. 1 to 8, Fig. 1 is a schematic diagram showing an application of a surgical robot system according to an embodiment of the present invention; Fig. 2 is a schematic diagram showing a surgical robot system according to an embodiment of the present invention; Fig. 3 is a flowchart of a control method for a surgical robot system according to an embodiment of the present invention; Figs. 4a-4c are schematic diagrams showing a tailing end of a surgical instrument according to an embodiment of the present invention; Fig. 5 is a flowchart of a control method for a surgical robot system with a unrecoverable malfunction according to an embodiment of the present invention; Fig. 6 is a flowchart of a control method for a surgical robot system with a recoverable malfunction according to an embodiment of the present invention; Fig. 7 is a flowchart of a control method for a surgical robot system according to another embodiment of the present invention; Fig. 8 is a schematic diagram showing the detection on the state of communicative connection according to an embodiment of the present invention.

An embodiment of the present invention provides a surgical robot system. Fig. 1 shows an application scenario of using the surgical robot system to perform surgical operations in an exemplary embodiment. The surgical robot system of the present invention has no particular limitation on the application environment. The surgical robot system includes a doctor-side console 10, an image trolley 20 and a patient-side operating table 30. The patient-side operating table 30 includes at least one mechanical arm 31. The mechanical arm 31 is configured to mount or connect a surgical instrument 40 or an endoscope. A operator manipulates the doctor-side console 10 to control the patient-side operating table 30 to drive the mechanical arm 31, so as to operate the surgical instrument 40 to perform surgical operations. Preferably, the surgical robot system further includes a hospital bed 50 and auxiliary equipment 60 (such as a sterile table, a ventilator or a detection device). Furthermore, the endoscope connected to the mechanical arm is used to obtain image information of surgical environments such as human tissues and organs, surgical instruments 40, blood vessels, and body fluids. The doctor-side console 10 also includes a display device configured to receive image information collected by the endoscope, that is, the above-mentioned image information of surgical environments.

Please refer to Fig. 2, a master manipulator is provided on the doctor-side console 10, and the main operation process of the surgical robot is that the operator (for example, a surgeon) performs remote operation at the doctor-side console 10 by using the master manipulator on the doctor-side console 10, so as to perform the minimally invasive surgical treatment on patients on the hospital bed 50. The master manipulator on the doctor-side console 10 is usually controlled manually by the surgeon and forms a master-slave mapping relationship with the surgical instrument 40 or the endoscope. During normal surgical operation, the operator controls the posture and clamping state of the tailing end of the surgical instrument 40 through master-slave teleoperation under the guidance of the image on the display device. Those skilled in the art can understand the structure and principle of the components of the surgical robot system according to the prior art.

Please refer to Fig. 3, based on the above-mentioned surgical robot system, this embodiment provides a control method for the surgical robot system, including:
Step S 1: configuring a corresponding response to malfunction according to the instrument information of different surgical instruments;
Step S2: monitoring a state of the surgical robot system, and determining the response to malfunction for a current surgical instrument according to the instrument information of the current surgical instrument when the surgical robot system is in a malfunction state; and,
Step S3: controlling the surgical robot system to perform a responsive operation according to the determined response to malfunction.

With such a configuration, according to the instrument information of different surgical instruments, the surgical robot system can determine different responses to malfunction and perform different responsive operations, which improves the safety of surgical operations performed by the surgical robot system.

Optionally, the instrument information at least includes state information of the surgical instrument, and the step of determining the response to malfunction for a current surgical instrument according to the instrument information of the current surgical instrument in Step S2 includes: determining the response to malfunction for a current surgical instrument according to the state information of the surgical instrument, wherein the response to malfunction includes: switching the surgical instrument to the state maintenance mode, and alternatively switching the surgical instrument not to the state maintenance mode but to the safety mode. In some embodiments, the surgical robot system further includes an instrument driver, which is preferably disposed on the patient-side operating table 30, and used to control the joints or motors that drive the surgical instruments. The instrument driver has enable information indicating whether it is enabled. The enable information indicates a disabling state or an enabling state. When the instrument driver is disabled, the instrument driver cannot control the joints or motors that drive the surgical instrument. The instrument driver does not maintain the position and posture of the surgical instrument, and does not maintain it in the clamping state, but switches to the safe mode. At this time, it is convenient for the operator to manually operate the surgical instrument and withdraw the surgical instrument. When the instrument driver is enabled, the instrument driver can control each joint or motor that drives the surgical instrument.

Optionally, the state information of the surgical instrument includes installation information indicating whether the surgical instrument is installed, and/or enabling information indicating whether the instrument driver is enabled. Preferably, when it is detected that the installation information indicates that the surgical instrument is installed and the enabling information indicates the enabling state, it is determined that the surgical instrument is in a normal operation process. At this time, the response to malfunction includes switching the surgical instrument to the state maintenance mode, in which the posture and the clamping state of the tailing end of the surgical instrument are maintained to avoid damage to the patient. If the installation information of the surgical instrument indicates that the surgical instrument is not installed, or when the enabling information indicates the disabling state, it is determined that the surgical robot is currently not in a normal operation, and the response to malfunction includes switching the surgical instrument to the safety mode, in which the instrument driver is disabled to facilitate the operator to address the malfunction.

Preferably, the instrument information also includes type information of the surgical instrument. The type information of the surgical instrument indicates a dual headed instrument or a single headed instrument. Please refer to Figs. 4a to 4c, which show a dual headed instrument 41, which includes a pitch joint 411, a yaw joint 412 (for example, left and right yaw), and a rotation joint 413. As shown in Fig. 4a, the posture of the dual headed instrument 41 is mainly determined by three joints that are a pitch joint 411, a yaw joint 412 and a rotation joint 413. The positions of all joints of the dual headed instrument 41 can be controlled in the state maintenance mode. As shown in Fig. 4b, the tailing end of the dual headed instrument 41 includes two clamping pieces 414, and the opening and closing of the two clamping pieces 414 are respectively controlled by one or two control motors. When the control motor controls the two clamping pieces 414 to close, the tailing end of the dual headed instrument 41 is in a clamping state, so as to clamp and pull tissue or clamp a suture needle, as shown in Fig. 4c. When the control motor controls the two clamping pieces 414 to open, the tailing end of the dual headed instrument 41 is in an open state, and the the object is no longer clamped by and released from it Therefore, when the clamping state of the surgical instrument is maintained, the control motor for the two clamping pieces 414 needs to be controlled. It should be understood that the dual headed instrument 41 shown in Figs. 4a to 4c is only an example and not a limitation to the dual headed instrument 41, and the dual headed instrument 41 may also be a needle holder commonly used in the art, an arc-shaped dual headed instruments with clamping function such as arc-shaped dual headed or duckbill forceps.

For example, single headed instruments can be instruments without clamping function such as electric hooks, and the adjustment and control of the posture of the tailing end can refer to those of the above-mentioned dual headed instruments. Since the tailing end of the single headed instrument has no clamping function and does not need to be opened and closed, there is no need to consider the adjustment and control of the clamping state of the single headed instrument. Thus, by acquiring the type information of the surgical instrument, the surgical robot system can specifically determine different responses to malfunction for the surgical instrument and perform different responsive operations when in a malfunction state.

Optionally, the state maintenance mode at least includes: maintaining the posture of the tailing end of the surgical instrument and/or maintaining a clamping state of the surgical instrument (the clamping state is associated with clamping posture and clamping force). Further, if the type information of the surgical instrument indicates a dual headed instrument, the state maintenance mode includes simultaneously maintaining the posture of the tailing end of the surgical instrument and maintaining the clamping state of the surgical instrument (for example, the state maintenance mode includes simultaneously keeping the posture of the tailing end of the surgical instrument unchanged and keeping the clamping force of the surgical instrument unchanged); if the type information of the surgical instrument indicates a single headed instrument, the state maintenance mode includes maintaining the posture of the tailing end of the surgical instrument. It can be understood that, in the control method for the surgical robot system according to this embodiment, in the case of a dual headed instrument that is configured for clamping, when the surgical robot system is in a malfunction state, simultaneously maintaining the posture of the tailing end of the surgical instrument and maintaining the clamping state of the surgical instrument enables the clamping state (that is, the clamping force) of the surgical instrument on vital tissues such as blood vessels or the prostate to be kept unchanged, which can effectively improve the safety of the surgical operation performed by the surgical robot system. In the case of a single headed instrument configured not for clamping, when the surgical robot system is in a malfunction state, only the posture of the surgical instrument is to be maintained to prevent the surgical instrument from uncontrolled movement which may cause a change in posture.

Further, the surgical robot system also includes a control device. Preferably, the control method for the surgical robot system described above can be perfomed by the control device. When a malfunction occurs in the surgical robot system, the control device determines the response to malfunction according to the instrument information of the surgical instrument, and directly controls the surgical instrument to perform posture maintenance and/or clamping state maintenance. In some other embodiments, the control device is also communicatively connected with the instrument driver, and the control device indirectly controls the joints or motors that drive the surgical instrument through the instrument driver.

Optionally, in an exemplary embodiment, the step of maintaining the posture of the tailing end of the surgical instrument includes: performing posture maintenance according to the current posture of the surgical instrument, or performing posture maintenance according to the posture command of the surgical instrument, or performing posture maintenance by tracking the current position of the surgical instrument in Cartesian coordinate system. Therefore, the posture of the tailing end of surgical instrument can be maintained in any one of the above three ways. Specifically, when the surgical robot is operating normally, the control device can acquire the current posture of the surgical instrument by controlling and driving the surgical instrument, and can drive the surgical instrument to adjust its posture by sending a posture command to the surgical instrument. In addition, the control device can also track the position of the surgical instrument in Cartesian coordinate system through other tracking devices. Therefore, when a malfunction occurs in the surgical robot system, the control device can control the surgical instrument to maintain its posture in any one of the above three ways. Further, when the surgical robot is in normal operation, the control device can also drive the clamping (that is, opening and closing) of the surgical instrument. Therefore, when a malfunction occurs in the surgical robot system, the control device can also control the surgical instrument to maintain its clamping state. In some other embodiments, the control device indirectly controls the surgical instrument to maintain the posture of the tailing end through the instrument driver in any one of the above three ways, or the control device indirectly maintains the clamping state of the surgical instrument through the instrument driver.

Optionally, the control method for the surgical robot system further includes:
Step S0: when the surgical robot system is in a malfunction state, based on the malfunction information received, determining a malfunction classification according to a predetermined regulation;
Step S4: after performing the responsive operation in Step S3, prompting recovery information corresponding to the malfunction classification according to the the malfunction classification. It should be noted that Step S0 here can be performed before Step S 1, or before Step S2, or after Step S2, but should be performed before Step S3.

When a malfunction occurs in the surgical robot system, the control device receives the malfunction information, and then determines the malfunction classification according to the predetermined regulation based on the type of the malfunction information such as driver failure information, motor code disk failure information, motor drive failure information and network communication failure information. For example, when the malfunction classification is classified into recoverable and unrecoverable malfunctions, the malfunction is to be determined as a recoverable malfunction or an unrecoverable malfunction. The recoverable malfunctions involve for example operations in an excessive speed and position deviation caused by arm collision, and the non-recoverable malfunctions are physical malfunctions such as motor disconnection, driver fault, code disc damage. According to different malfunction classifications, the corresponding recovery information is prompted for the operator to perform corresponding operations.

Please refer to Fig. 5, preferably, prompting recovery information corresponding to the unrecoverable malfunction includes prompting restart information for the operator to perform a restart operation. When an unrecoverable malfunction occurs in the surgical robot system, the operator can generally perform a recovery operation only by powering off and restarting the system. Therefore, a restart imformation is prompted. Please refer to Fig. 6, preferably, prompting recovery information corresponding to the recoverable malfunction includes prompting a fault code for the operator to perform a cancelling operation. When a recoverable malfunction occurs in the surgical robot system, the operator can generally perform a recovery operation by clearing the fault code. For example, the fault code can be prompted in the interactive software, and the operator can address the malfunction by canceling the fault code.

Optionally, in an example, the surgical robot system further includes a prompting component including for example at least one of a signal light, a buzzer, and a display screen/interactive software for displaying interactive information on the mechanical arm. In Step S3, an alarm or prompt information may be displayed through the prompting component to prompt the operator. Table 1 exemplarily shows circumstances where different prompts are made for different malfunction classifications. It should be understood that the following table is used to show an example for the prompts made for malfunction classifications rather than a limitation thereto.

**Table 1**

| Malfunction Classification | Signal Light | Buzzer | Interactive Software |
|---|---|---|---|
| Recoverable Malfunction | The yellow lights on a single mechanical arm or multiple mechanical arms are flashing (slow flashing) | Alarm with a medium frequency | Prompt fault code in image and text |
| Unrecoverable Malfunction | The red lights on a single mechanical arm or multiple mechanical arms are flashing (fast flashing) | Alarm with a high frequency | Prompt restart information in image and text |

Please refer to Figs. 7 and 8, the communicative connection between the control device 71 and the instrument driver 72 of the surgical robot system relies on a verification mechanism, which for example performs communication verification through real-time beat wave signals 70 to detect whether the state of the communicative connection is normal. Preferably, when the state of the communicative connection is abnormal, for example, when a communication failure such as network disconnection occurs, the above-mentioned control method for the surgical robot system can be performed by the instrument driver 72, so as to further improve safety. Optionally, in the surgical robot system, the control device 71 indirectly controls each joint or motor that drives the surgical instrument through the instrument driver 72. Specifically, the control device 71 determines the response to malfunction according to the instrument information of the surgical instrument, then sends the response to malfunction to the device driver 72 in real time. Once the device driver 72 detects that the state of the communicative connection with the control device 71 is abnormal, a responsive operation is performed according to the response to malfunction sent from the control device at the previous moment. Of course, in some other embodiments, the instrument driver 72 can itself determine the response to malfunction according to the instrument information of the surgical instrument, so as to independently decide how to maintain the surgical instrument, and execute the response to malfunction. For example, when the network is disconnected, the device driver 72 can independently determine the responsive operation, and does not need to perform the responsive operation according to the response to malfunction sent from the control device at the previous moment.

With such a configuration, when the communication between the control device 71 and the instrument driver 72 is normal, the control device 71 protects the the entire surgical robot system for safety, and maintains the posture and clamping state of the surgical instrument. When the communication between the control device 71 and the instrument driver 72 is abnormal, the instrument driver 72 can independently protect the surgical instrument for safety, and maintains the posture and clamping state of the surgical instrument. When there is a communication failure such as disconnection of the network, and the instrument driver 72 loses connection with the control device 71, the instrument driver 72 can still independently maintain the posture and clamping state of the tailing end of the surgical instrument, further improving the safety of the surgical robot system.

This embodiment also provides a readable storage medium storing a program, and when the program is executed, the control method for the surgical robot system as described above is implemented. The readable storage medium can be integrated in the surgical robot system, for example integrated in the control device or the instrument driver, or can be independently provided. Further, the surgical robot system according to this embodiment is configured such that, when the surgical robot system is in a malfunction state, the control device performs a responsive operation according to the control method for the surgical robot system as described above. Optionally, according to the above-mentioned control method for the surgical robot system, the state of the communicative connection between the instrument driver and the control device is detected, and the device driver or the control device is selected to perform a responsive operation, depending on whether the state of the communicative connection is normal or not.

In conclusion, in the control method for the surgical robot system, the readable storage medium and the surgical robot system according to the present invention, the control method for the surgical robot system includes: configuring a corresponding response to malfunction according to the instrument information of different surgical instruments; monitoring a state of the surgical robot system, and determining the response to malfunction for a current surgical instrument according to the instrument information of the current surgical instrument when the surgical robot system is in a malfunction state; and controlling the surgical robot system to perform a responsive operation according to the determined response to malfunction. With such a configuration, according to the instrument information of different surgical instruments, the surgical robot system can determine different responses to malfunction and perform corresponding responsive operations, which improves the safety of surgical operations performed by the surgical robot system.

The above is only for describing preferred embodiments of the present invention, and is not intended to limit the scope of the present invention. Any changes and modifications made by those skilled in the art according to the above disclosure are all within the protection scope of the appended claims.

## Claims

1. A control method for a surgical robot system, comprising:
Step S 1: configuring a corresponding response to malfunction according to instrument information of different surgical instruments;
Step S2: monitoring a state of the surgical robot system, and determining the response to malfunction for a current surgical instrument according to the instrument information of the current surgical instrument when the surgical robot system is in a malfunction state; and,
Step S3: controlling the surgical robot system to perform a responsive operation according to the determined response to malfunction.

2. The control method for the surgical robot system according to claim 1, wherein the instrument information comprises state information of the surgical instrument, and wherein determining the response to malfunction for a current surgical instrument according to the instrument information of the current surgical instrument in Step S2 comprises:
determining the response to malfunction for a current surgical instrument according to the state information of the surgical instrument;
wherein the response to malfunction comprises: switching the surgical instrument to a state maintenance mode, and switching the surgical instrument not to the state maintenance mode but to a safety mode.

3. The control method for the surgical robot system according to claim 2, wherein the instrument information further comprises type information of the surgical instrument; the type information of the surgical instrument indicates a dual headed instrument or a single headed instrument; if the type information of the surgical instrument indicates a dual headed instrument, the state maintenance mode comprises simultaneously maintaining a posture of a tailing end of the surgical instrument and a clamping state of the surgical instrument; if the type information of the surgical instrument indicates a single headed instrument, the state maintenance mode comprises maintaining the posture of the tailing end of the surgical instrument

4. The control method for the surgical robot system according to claim 3, wherein maintaining the posture of the tailing end of surgical instrument comprises at least one of the following:
performing posture maintenance according to a current posture of the surgical instrument, or
performing posture maintenance according to a posture command of the surgical instrument, or
performing posture maintenance by tracking a current position of the surgical instrument in Cartesian coordinate system.

5. The control method for the surgical robot system according to claim 2, wherein the state information of the surgical instrument comprises installation information indicating whether the surgical instrument is installed, and/or enabling information indicating whether an instrument driver is enabled.

6. The control method for the surgical robot system according to claim 5, wherein
when it is detected that the installation information indicates that the surgical instrument is installed and the enabling information indicates an enabling state, the response to malfunction comprises switching the surgical instrument to the state maintenance mode;
when it is detected that the installation information indicates that the surgical instrument is not installed, or the enabling information indicates a disabling state, the response to malfunction comprises switching the surgical instrument to the safety mode.

7. The control method for the surgical robot system according to claim 1, further comprising:
Step S0: determining a malfunction classification according to a predetermined regulation based on a malfunction information received when the surgical robot system is in a malfunction state;
Step S4: after performing the responsive operation in Step S3, prompting recovery information corresponding to the malfunction classification according to the malfunction classification.

8. The control method for the surgical robot system according to claim 7, wherein the malfunction classification comprises recoverable malfunction and unrecoverable malfunction;
prompting recovery information corresponding to the recoverable malfunction comprises prompting a fault code for an operator to perform a cancelling operation;
prompting recovery information corresponding to the unrecoverable malfunction comprises prompting restart information for the operator to perform a restart operation.

9. The control method for the surgical robot system according to any one of claims 1 to 8, further comprising:
detecting a state of a communicative connection between an instrument driver and the control device; if the state of the communicative connection is abnormal, the instrument driver performs the control method for the surgical robot system according to any one of claims 1 to 8; if the state of the communicative connection is normal, the control device performs the control method for the surgical robot system according to any one of claims 1-8.

10. A readable storage medium, storing a program, wherein
when the program is executed, the control method for the surgical robot system according to any one of claims 1-9 is performed.

11. A surgical robot system, comprising: a patient-side operating table and a control device;
wherein the patient-side operating table comprises a mechanical arm to which a surgical instrument is mounted or connected; the control device is communicatively connected to the surgical instrument;
wherein the surgical robot system is configured such that:
when the surgical robot system is in a malfunction state, the control device performs a responsive operation according to the control method for the surgical robot system according to any one of claims 1 to 8; or
according to the control method for the surgical robot system according to claim 9, the state of the communicative connection between an instrument driver and the control device is detected, and the device driver or the control device is selected to perform a responsive operation, depending on whether the state of the communicative connection is normal or abnormal.
